# FASCICULE DE BREVET EUROPEEN

(11) **EP 0 630 287 B1**
(45) Date de publication et mention de la délivrance du brevet: **09.10.1996**
(21) Numéro de dépôt: 93918737.3
(22) Date de dépôt: 10.03.1993
(51) Int. Cl.: B01J 13/08, A61K 9/50

(54) **MICROCAPSULES A PAROIS EN POLYSACCHARIDES CONTENANT DES FONCTIONS ALCOOLS PRIMAIRES, ET COMPOSITIONS EN CONTENANT**
MIKROKAPSELN MIT WÄNDEN AUS PRIMÄREN ALKOHOLFUNKTIONEN ENTHALTENDEN POLYSACCHARIDEN, UND ZUSAMMENSETZUNGEN DARAUS
MICROCAPSULES WITH PRIMARY ALCOHOL FUNCTIONS CONTAINING POLYSACCHARIDE WALLS AND COMPOSITIONS CONTAINING THEM

(30) Priorité: 11.03.1992 FR 9202912
(43) Date de publication de la demande: 28.12.1994
(73) Titulaire: COLETICA, 69007 Lyon (FR)
(72) Inventeur: PERRIER, Eric, Jean-Luc, F-38200 Vienne (FR); BUFFEVANT, Chantal, Marie, F-69390 Millery (FR)
(74) Mandataire: Portal, Gérard
(86) Numéro de dépôt international: FR9300237
(87) Numéro de publication internationale: WO9317784

(56) Documents cités:
- EP-A- 0 273 823
- EP-A- 0 277 741
- EP-A- 0 339 866
- EP-A- 0 481 240
- WO-A-89/00419
- DE-B- 1 238 890
- US-A- 3 398 015
- WO 88/08747

## Description

La présente invention concerne essentiellement l'utilisation de polysaccharides ayant des fonctions alcools primaires pour la fabrication de microcapsules ou microsphères, des microcapsules ou microsphères ainsi réalisées, un procédé de fabrication de telles microcapsules ou microsphères et des compositions cosmétiques, pharmaceutiques ou alimentaires en contenant.

Le déposant a déjà décrit dans le document FR-A-2 642 329 l'utilisation de solutions d'atélocollagène et de glycosaminoglycannes pour la fabrication de microcapsules, les microcapsules elles-mêmes, leur procédé de fabrication ainsi que des compositions cosmétiques, pharmaceutiques ou alimentaires en contenant.

Ces microcapsules à paroi mixte atélocollagène-glycosaminoglycanne donnent entière satisfaction.

Cependant, il peut être très intéressant, dans certains cas, d'utiliser des microcapsules ou microsphères réalisées à partir de matériaux issus du règne végétal ou obtenus par fermentation microbienne.

Il a également été proposé la fabrication de microcapsules par polymérisation interfaciale de monomère synthétique, plus particulièrement sur des diamines de synthèse de manière à former des fonctions amides de la même manière que lors de la synthèse chimique du Nylon, ce qui permet de constituer une enveloppe résistante autour de la capsule. Le coeur de la capsule n'intervient pas dans la réaction de polymérisation et peut être constitué de protéine (FR-76 34482 = FR 2,370,520 et FR-79 2012 = FR 2,415,593) de polysaccharide (US-4 322 311).

Il a étalement été proposé la fabrication de capsules à base de protéine réticulée par des dérivés de diacide (FR-A-1 415 039) ou par du glutaraldéhyde (WO-82/00660).

Il a également été proposé de fabriquer des capsules dont la paroi est à base de polysaccharides réticulés ioniquement par des cations mono- di- ou trivalents (US-A-4 495 288) ou à l'aide de molécules polycationiques de haut poids moléculaire (EP-A1-0 301 777; US-A-4 407 957; US-A-4 391 309).

Il a également été proposé de fabriquer des capsules à base de polysaccharides aminés, par exemple le chitosane, réticulés avec des agents bifonctionnels comme le glutaraldéhyde (WO-82/00660).

La présente invention a pour but de résoudre le nouveau problème technique consistant en la fourniture d'une solution permettant de fabriquer des microcapsules ou des microsphères à base d'une matière première d'excellente biocompatibilité, de biodégradabilité totale, d'assimilation complète, d'innocuité totale, provenant d'un matériau biologique renouvelable, et pouvant être présente non seulement dans le règne animal, mais aussi dans le règne végétal et pouvant être aussi produite par fermentation microbienne.

Ce nouveau problème technique est résolu pour la première fois d'une manière satisfaisante, peu coûteuse, utilisable à l'échelle industrielle, notamment alimentaire, cosmétique ou pharmaceutique.

Ainsi, selon un premier aspect, la présente invention concerne l'utilisation de polysaccharides ayant des fonctions alcools primaires pour la fabrication de microcapsules ou de microsphères qui, de préférence, contiennent un principe actif, notamment alimentaire, cosmétique ou pharmaceutique, par réticulation interfaciale des fonctions d'alcool primaire dudit polysaccharide avec un agent de réticulation interfaciale constitué par un dichlorure d'acide ou un anhydride d'acide. Dans ce cadre, les polysaccharides à fonction alcool primaire ont de préférence un poids moléculaire supérieur à 5 000 daltons. En outre, ces polysaccharides à fonction alcool primaire présentent avantageusement de 1 à 4 fonctions alcools primaires par motif diosidique.

Selon un deuxième aspect, la présente invention concerne aussi des microcapsules ou des microsphères caractérisées en ce qu'elles comprennent une paroi en polysaccharide ayant des fonctions alcools primaires réticulées
par réticulation interfaciale à l'aide d'un agent de réticulation constitué par un dichlorure d'acide ou un anhydride d'acide.

Selon un mode de réalisation avantageux, le polysaccharide à fonction alcool primaire précité présente un poids moléculaire supérieur à 5 000 daltons.

Selon un mode de réalisation préféré, le polysaccharide précité comprend de 1 à 4 fonctions alcools primaires par motif diosidique.

Selon un mode de réalisation particulier, les polysaccharides à fonction alcool primaire précités sont choisis parmi le groupe consistant en :
(a) les polysaccharides naturels ou obtenus par fermentation, en particulier :
   - les galactomannanes, par exemple galactomannanes issus de guar tels que Viscogum® (SANOFI), ou de caroube tels que ceux commercialement disponibles sous le nom de Lygomme® (SANOFI) ou Meypro-Fleur®, ou Meyprodyn® (MEYHALL);
   - les carraghénanes tels que ceux extraits des algues rouges comme ceux commercialement disponibles sous le nom de Satiagel® ou Satiagum® (SANOFI);
   - les glucomannanes, tels que ceux issus du KONJAC, tels que ceux commercialement disponibles sous le nom de Nutricol® (FMC Corporation) ou de Propol® (SHIMITZU);
   - les gommes naturelles;
   - l'amylose ou l'amylopectine et leurs mélanges qui sont extraits des végétaux ou de leurs fruits comme par exemple du blé, du mais, du pois ou de la pomme de terre;
   - les polysaccharides à fonction alcool primaire issus de fermentation comme le Xanthane (KELKO), le Gellane® (KELKO) ou le Curdlane® (TAKEDA).
(b) Les polysaccharides chimiquement modifiés par fixation ou création d'alcool primaire sur le polymère.

Il peut en effet être très avantageux d'introduire des groupements alcools primaires jouant le rôle de bras espaceur espaçant des groupements alcools primaires du squelette du polymère pour les rendre plus aisément accessibles, notamment pour une éventuelle réaction avec l'agent de réticulation comme cela sera décrit plus loin dans le cadre du procédé de fabrication.

Comme groupement alcool primaire on peut citer par exemple un radical hydroxyalkyle en C₁-C₃₀, en particulier en C₁-C₆.

Des exemples de polysaccharides chimiquement modifiés sont des celluloses au moins partiellement éthérifiées par des radicaux alkylhydroxy, en particulier par éthérification avec une ou plusieurs fonctions hydroxy de la molécule cellulosique avec un oxyde d'alkylène correspondant comme cela est bien compréhensible à un homme de l'art. Des agents d'éthérification particulièrement intéressants, aisés à mettre en oeuvre sont les oxydes de méthylène, d'éthylène, de propylène, de butylène. En outre, des polysaccharides chimiquement modifiés de ce type sont commercialement disponibles comme par exemple les hydroxyéthylcelluloses de type Natrosol commercialisées par la Société Aqualon. On comprend ainsi que l'invention n'est nullement limitée à une liste particulière de polysaccharides chimiquement modifiés mais qu'elle englobe tous les polyssacharides chimiquement modifiés pour rendre plus aisément accessibles des fonctions alcools primaires.

Selon un mode de réalisation avantageux, les microcapsules ou microsphères précitées contiennent un actif hydrosoluble, hydrodispersible, insoluble ou liposoluble, soit présent dans la paroi des microcapsules ou microsphères, soit encore encapsulé à l'intérieur desdites microcapsules ou microsphères. Il peut s'agir d'un principe actif alimentaire, cosmétique ou pharmaceutique.

Selon un troisième aspect, la présente invention fournit également un procédé de fabrication des microcapsules ou des microsphères précitées, caractérisé en ce qu'on disperse ou dissout un polysaccharide ayant des fonctions alcools primaires en phase aqueuse, en obtenant une phase aqueuse contenant en dispersion ou à l'état dissous ledit polysaccharide, puis cette phase aqueuse contenant le polysaccharide est mise en contact avec une phase organique non miscible à l'eau contenant un agent de réticulation constitué par un dichlorure d'acide ou un anhydride d'acide pour réaliser une réticulation interfaciale entre les fonctions alcools primaires du polysaccharide pour une durée suffisante pour obtenir lesdites microsphères ou microcapsules à paroi comprenant ledit polysaccharide ayant des fonctions alcools primaires réticulés par le dichlorure d'acide ou un anhydride d'acide, en formant ainsi des liaisons ester, lesdites microsphères ou microcapsules sont ensuite séparées du milieu réactionnel par tout moyen de séparation.

Selon une caractéristique préférée du procédé selon l'invention, l'agent de réticulation est choisi parroi le dichlorure d'acide téréphtalique, le dichlorure d'acide phtalique, le dichlorure d'acide sébacique, le dichlorure d'acide succinique, le dichlorure ou le trichlorure d'un acide tricarboxylique comme l'acide citrique ou un di-anhydride d'acide comme le di-anhydride succinique.

Selon un autre mode particulier de réalisation du procédé selon l'invention, la phase organique insoluble dans l'eau ou hydrophobe comprend une huile végétale, un ester d'acide gras, une huile minérale, un silicone ou un solvant organique apolaire, en particulier l'hexane, le cyclohexane ou le chloroforme.

Selon un autre mode de réalisation particulièrement avantageux de l'invention, le polysaccharide utilisé selon l'invention contenant des fonctions alcools primaires présente un poids moléculaire supérieur à 5 000 daltons.

Selon une autre variante de réalisation particulière, la concentration en polysaccharide à fonction alcool primaire est comprise entre 0,2% et 30% en poids de la phase aqueuse.

Selon une autre variante de réalisation particulièrement avantageuse du procédé selon l'invention, la phase aqueuse est une phase aqueuse alcaline, c'est-à-dire dont le pH est un pH alcalin, donc supérieur à 7. Un domaine de pH préféré est un domaine de pH compris entre environ 7,1 et environ 10.

Comme base pour amener la phase aqueuse à un pH basique, on peut utiliser un tampon ammoniaque, borate, phosphate ou carbonate.

Selon une variante de réalisation particulièrement avantageuse, la phase aqueuse, de préférence basique précitée, est émulsionnée, éventuellement à l'aide d'un agent émulsionnant dans une phase huileuse. Comme agent émulsionnant, on peut utiliser par exemple les produits commercialisés par ICI tels que les "SPAN®", les "TWEEN®", les "BRIJ®", les "ARLACEL®".

Les proportions en poids de l'agent réticulant à mettre en oeuvre par rapport au poids de la solution polysaccharidique utilisée varient entre 20 et 60%, mais sont préférentiellement situées entre 30 et 45%.

Le temps de réticulation interfaciale peut varier dans de larges limites et dépend de la matière première utilisée, de l'agent de réticulation, de la nature des phases ainsi que de la dimension des microcapsules ou des microsphères recherchées. Ce temps de réticulation interfaciale est relativement long et variera en général entre 15 min et 24 h et en particulier voisin de 90 min.

Selon une autre variante de réalisation avantageuse, on peut mélanger intimement avec la phase aqueuse contenant le polysaccharide un actif hydrosoluble, hydrodispersible, insoluble ou liposoluble. Cet actif peut être en particulier à usage alimentaire, cosmétique ou pharmaceutique.

Lorsqu'il s'agit d'un principe actif liposoluble, on peut utiliser en outre un agent stabilisateur d'émulsion tel qu'un colloïde, une macromolécule, un agent émulsionnant synthétique ou naturel.

Selon une variante de réalisation particulière, lorsque le polysaccharide n'est pas soluble à la température ambiante dans la phase aqueuse, de préférence alcaline, on peut procéder à une étape de chauffage pendant une durée suffisante pour dissoudre totalement le polysaccharide et on refroidit la phase aqueuse à la température ambiante. En général, un chauffage à environ 90°C pendant 1 h est suffisant pour dissoudre totalement le polysaccharide.

On peut réaliser la séparation des sphères ou capsules obtenues après réticulation par tout moyen approprié, notamment par décantation naturelle ou par centrifugation.

On peut également effectuer un ou plusieurs lavages de ces sphères ou capsules.

Selon une variante de réalisation particulière, on peut aussi ensuite mettre les sphères ou capsules en suspension dans un gel, une phase silicone ou une phase liquide, en fonction des applications envisagées.

Les sphères ou capsules à base de polysaccharide à fonction alcool primaire réticulé selon l'invention peuvent être utilisées dans des formulations alimentaires, cosmétiques ou pharmaceutiques.

Ainsi, selon un quatrième aspect, la présente invention couvre aussi une composition alimentaire, cosmétique ou pharmaceutique, caractérisée en ce qu'elle comprend des microcapsules ou des microsphères à paroi de polysaccharide ayant des fonctions alcools primaires réticulées par un agent de réticulation qui, de préférence, contiennent au moins en partie une substance présentant un intérêt alimentaire, pharmaceutique ou cosmétique.

Selon un cinquième aspect, la présent invention couvre encore un procédé de préparation d'une composition alimentaire, pharmaceutique ou cosmétique, caractérisé en ce qu'on utilise au moins en partie des microcapsules ou des microsphères à paroi en polysaccharide à fonctions alcools primaires réticulées par un agent de réticulation interfaciale constitué par un dichlorure d'acide ou un anhydride d'acide en formant ainsi une liaison ester, lesdites microcapsules ou microsphères, de préférence, contiennent au moins en partie une substance présentant un intérêt alimentaire, cosmétique ou pharmaceutique, éventuellement dans un excipient acceptable en alimentaire, en cosmétique ou en pharmaceutique.

La dimension des capsules ou des sphères sera généralement comprise entre 5 µm et 900 µm. Le dimension des capsules ou sphères peut être réglée en réglant la vitesse d'agitation lors de la réticulation interfaciale, comme cela est bien connu à l'homme de l'art.

Dans la description et les revendications, les termes microcapsules ou microsphères sont utilisés indifféremment de manière équivalente pour désigner des particules comportant à l'intérieur un espace permettant l'encapsulation d'un principe actif, en particulier dans une phase aqueuse ou non.

Les capsules ou sphères selon la présente invention, à base de polysaccharide contenant des fonctions alcools primaires, présentent une biocompatibilité excellente puisque les polysaccharides utilisés ne sont pas cytotoxiques, une biodégradabilité totale puisque les polysaccharides utilisés sont facilement biodégradables, par exemple par des amylases bactériennes ; leur assimilation est complète car les résidus de dégradation sont des oses simples qui peuvent être utilisés directement par la cellule comme source d'énergie ; leur toxicité est quasiment nulle puisque des tests d'irritation orale, cutanée, occulaire qui ont été réalisés par les inventeurs ont démontré leur totale innocuité in vitro et in vivo chez l'animal.

D'autre part, les polysaccharides ayant des fonctions alcools primaires sont obtenus à partir de matériaux biologiques renouvelables. Ils sont largement répandus dans la nature puisqu'on les trouve non seulement chez les animaux, mais aussi dans les micro-organismes ainsi que dans les végétaux supérieurs et les algues où ils représentent plus de 75% de leur poids sec.

De plus, les polysaccharides permettent un excellent piégeage des principes actifs, y compris ceux ayant une faible masse moléculaire, soit dans la paroi, soit par encapsulation à l'intérieur des sphéres ou capsules.

L'invention permet d'utiliser des polysaccharides à fonction alcool primaire provenant en particulier de fermentation microbienne ou de végétaux, ce qui permet de s'affranchir de l'utilisation de produits extraits du règne animal.

L'invention permet encore d'utiliser des polysaccharides chimiquement modifiés contenant des fonctions alcools primaires plus aisément accessibles grâce à l'introduction de bras espaceur comme précédemment décrit. Ces groupements alcools primaires peuvent être rajoutés à des fonctions alcools déjà existantes, par éthérification, ou bien être complètement créés sur la structure du polymère.

Dans le cas de la présente invention, par usage pharmaceutique, on entend aussi bien l'usage pharmaceutique dans le cadre de la médecine animale ou de la médecine humaine.

D'autres buts, caractéristiques et avantages de l'invention apparaîtront clairement à la lumière de la description explicative qui va suivre faite en référence à plusieurs exemples de l'invention donnés simplement à titre d'illustration et qui ne sauraient donc en aucune façon limiter la portée de l'invention. Dans les exemples, les pourcentages sont donnés en poids sauf indication contraire.

### Exemple 1 : Sphères polysaccharidiques standards (5 à 900 micromètres)

### 1. Une préparation de polysaccharide de caroube est préparée comme suit :

6,4 kg de farine de caroube (Lygomme, SANOFI) sont mis en suspension dans 400 kg d'eau déminéralisée froide. La préparation est chauffée à 90°C pendant 1 h afin d'obtenir une solvatation complète des chaînes polysaccharidiques. La solution très visqueuse obtenue est mise a refroidir jusqu'à température ambiante. 20 kg de NaHCO₃ sont alors ajoutés à la préparation sous forte agitation. Après dissolution complète, le pH se stabilise à une valeur comprise entre 8 et 8,4.

### 2. Préparation de l'émulsion

50 kg de préparation précédemment décrite sont émulsionnés dans une phase huileuse telle que 150 l de DRAGOXAT® (DRAGOCO) et 10,5 l de SPAN® 85 (ICI).

Suivant la taille des sphères désirée, l'agitation est effectuée à l'aide d'un agitateur mécanique (taille comprise entre 100 et 900 µm), ou d'un Ultra-turax® tournant à 7 000 tr/min (taille comprise entre 5 et 100 µm).

### 3. Réticulation des sphères formées

Une phase huileuse comprenant 200 l de DRAGOXAT® et 18 kg de dichlorure d'acide téréphtalique est préparée sous agitation. Après dissolution complète du dichlorure d'acide, la phase huileuse est rajoutée sous agitation, à l'émulsion décrite en (2).

L'ensemble est laissé sous agitation pendant 90 min, afin de provoquer une polymérisation entre le diacide activé d'une part, et les fonctions alcools du polysaccharide d'autre part. Cette réaction est favorisée en milieu basique, et des temps de réaction relativement longs sont nécessaires pour que la réticulation soit suffisamment intense.

### 4. Présentation des sphères

Après réaction, les sphères sont récupérées par décantation ou par centrifugation, suivant leur taille.

Elles sont lavées plusieurs fois, puis sont mises en suspension dans un gel ou une solution, organique ou minéral.

Elles peuvent être utilisées telles quelles en alimentaire, cosmétique ou pharmaceutique.

### Exemple 2 : Sphères polysaccharidiques colorées

Au cours de l'étape (2) de l'exemple 1, 0,75 kg de pigment nacré (FLAMENCO) est ajouté aux 50 kg de la préparation polysaccharidique.

Toutes les autres étapes sont identiques par ailleurs. On peut également encapsuler de nombreux pigments insolubles et la couleur des sphères est modifiable à souhait.

### Exemple 3 : Sphères polysaccharidiques contenant un actif aqueux

Au cours de l'étape (1), 8,8 kg de farine de caroube sont mis en suspension dans 400 kg d'eau déminéralisée.

Au cours de l'étape (2), on rajoute à 30 kg de la préparation décrite en (1) 20 kg d'une solution aqueuse contenant le principe actif. Citons pour exemple un extrait aqueux de GINKGO BILOBA, ou d'ORCHIDEE ou de tout autre végétal.

Après mélange intime des deux composés, la préparation est émulsionnée, les sphères sont réticulées puis lavées comme décrit dans l'exemple 1.

### Exemple 4 : Sphères polysaccharidiques contenant une poudre hydrosoluble ou un réactif sous forme insoluble

Comme pour l'exemple 3, une préparation de farine de caroube est réalisée, puis à 45 kg de cette préparation sont ajoutés 1 à 10 kg d'un principe actif pulvérulent hydrosoluble ou insoluble.

Citons comme exemple des actifs ainsi encapsulés, des extraits végétaux (ALBAN, MULLER, WILLIAM RANSOM), des principes actifs pulvérulents animaux ou végétaux, des plantes ou des algues micronisées, ou tout autre extrait sec.

Après mélange intime, la préparation est émulsionnée, les sphères sont réticulées puis lavées comme décrit dans l'exemple 1.

### Exemple 5 : Sphères polysaccharidiques contenant un actif huileux ou liposoluble

Après préparation du gel de polysaccharide comme décrit dans l'exemple 1, une première émulsion huile-dans-eau est réalisée, en présence ou non d'un agent stabilisateur d'émulsions (colloïdes, macromolécules, émulsionnants), où la phase huileuse est la phase contenant le principe actif, et où la phase aqueuse est le gel polysaccharidique.

Ainsi par exemple, à 30 kg de la préparation polysaccharidique sont ajoutés 1 à 20 kg d'une phase huileuse comme le palmitate de vitamine A ou l'acétate de vitamine E.

Après agitation vigoureuse par tout moyen mécanique susceptible de réaliser une très fine émulsion (turbine, Ultra-turax, ultra-sons), le mélange intime est émulsionné, les sphères sont réticulées puis lavées comme décrit dans l'exemple 1.

Dans tous les exemples précédents, il a été utilisé les mots sphères mais ceux-ci incluent aussi les capsules. D'autre part, les sphères ou capsules obtenues peuvent être utilisées telles quelles en alimentaire, cosmétique ou pharmaceutique. Dans ce cadre de préférence, un principe actif est également encapsulé comme cela est par exemple décrit aux exemples 3 à 5.

Par ailleurs, les exemples ont été donnés en utilisant comme polysaccharide une farine de caroube exempte de galactomannanes. On peut aussi utiliser comme polysaccharides des carraghénanes, des glucomannanes, des gommes naturelles, de l'amylose ou amylpectine ou des polysaccharides issus de fermentation. Cependant, il est préféré d'utiliser un galactomannane, en particulier le caroube, car on obtient de très bons rendements avec une procédure simple, utilisable à l'échelle industrielle, notamment alimentaire, cosmétique ou pharmaceutique.

On peut également utiliser des polysaccharides chimiquement modifiés contenant des fonctions alcools primaires espacées de la chaîne de polymère peur être plus aisément accessibles notamment vis-à-vis d'une éventuelle réaction avec un agent de réticulation, pour la formation des microsphères ou microcapsules selon la présente invention. On peut ainsi utiliser des dérivés hydroxyalkylés de polysaccharides, en particulier des dérivés hydroxyalkylés en C₁-C₃₀ et notamment des dérivés hydroxyalkylés en C₁-C₃₀ de cellulose. L'exemple suivant constitue un exemple de réalisation de cette catégorie.

### Exemple 6 : Sphères polysaccharidiques à partir de polysaccharides chimiquement modifiés

On procède comme décrit à l'exemple 1, si ce n'est que l'étape 1 de préparation utilise un polysaccharide chimiquement modifié disponible dans le commerce tel qu'une hydroxyéthylcellulose du type Natrosol 250® commercialisée par la Société Aqualon, en lieu et place d'un polysaccharide de caroube. La préparation du polysaccharide est la suivante :

on met en suspension 12 kg d'hydroxyéthylcellulose disponible dans le commerce sous le nom commercial Natrosol 250® de chez Aqualon dans 400 kg d'eau froide déminéralisée, en obtenant ainsi une concentration de 3% en poids de polysaccharide chimiquement modifié. La préparation est alors chauffée à 40°C pendant 1 h sous agitation afin d'obtenir une préparation très homogène. Après refroidissement jusqu'au voisinage de la température ambiante, on ajoute 20 kg de NaHCO₃ sous forte agitation. Après dissolution complète, le pH se stabilise à une valeur comprise entre 8 et 8,4.

Le reste de la préparation est identique à l'exemple 1.

Ainsi, l'invention comprend tous les moyens constituant des équivalents techniques des moyens décrits ainsi que leurs diverses combinaisons.

## Revendications

1. Utilisation de polysaccharides ayant des fonctions alcools primaires pour la fabrication de microcapsules ou microsphères par réticulation interfaciale des fonctions alcools primaires dudit polysaccharide avec un agent de réticulation interfaciale constitué par un dichlorure d'acide ou un anhydride d'acide.

2. Microcapsules ou microsphères, caractérisées en ce qu'elles comprennent un paroi en polysaccharide ayant des fonctions alcools primaires réticulées par réticulation interfaciale par un agent de réticulation interfaciale constitué par un dichlorure d'acide ou un anhydride d'acide.

3. Microcapsules ou microsphères selon la revendication 2, caractérisées en ce que le polysaccharide précité présente un poids moléculaire supérieur à 5 000 daltons.

4. Microcapsules ou microsphères selon la revendication 2 ou 3, caractérisées en ce que le polysaccharide précité comprend de 1 à 4 fonctions alcools primaires par motif diosidique.

5. Microcapsules ou microsphères selon l'une des revendications 2 à 4, caractérisées en ce que le polysaccharide précité est choisi parmi un polysaccharide naturel, par exemple les galactomannanes, les carraghénanes, les glucomannanes, les gommes naturelles, l'amylose ou l'amylopectine et leurs mélanges, les polysaccharides issus de fermentation ; ou un polysaccharide chimiquement modifié comprenant des groupements alcools primaires espacés de la chaîne de polymère plus aisément accessibles notamment à une éventuelle réaction avec un agent de réticulation, en particulier des dérivés hydroxyalkyles en C₁-C₃₀ de polysaccharides.

6. Microcapsules ou microsphères selon l'une des revendications 2 à 5, caractérisées en ce qu'elles contiennent un principe actif alimentaire, cosmétique ou pharmaceutique.

7. Procédé de fabrication de microcapsules ou microsphères, caractérisé en ce qu'il comprend les étapes suivantes :
on disperse ou dissout en phase aqueuse un polysaccharide ayant des fonctions alcools primaires en obtenant une phase aqueuse contenant en dispersion ou à l'état dissous ledit polysaccharide, on met en contact ladite phase aqueuse avec une phase organique non miscible à l'eau et contenant un agent de réticulation interfaciale constitué par un dichlorure d'acide ou un anhydride d'acide pour réaliser une réticulation interfaciale desdites fonctions alcools primaires du polysaccharide pendant une durée suffisante pour obtenir des microsphères ou microcapsules à paroi comprenant ledit polysaccharide ayant des fonctions alcools primaires réticulées par le dichlorure d'acide ou l'anhydride d'acide en formant ainsi des liaisons ester, et lesdites microsphères ou microcapsules sont ensuite séparées du milieu réactionnel par tout moyen de séparation.

8. Procédé selon la revendication 7, caractérisé en ce qu'on utilise une phase aqueuse alcaline, ayant un pH supérieur à 7, en particulier compris entre environ 7,1 et environ 10.

9. Procédé selon l'une des revendications 7 à 8, caractérisé en ce que le polysaccharide précité est présent dans la phase aqueuse à une concentration comprise entre 0,2% et 30% en poids de ladite phase aqueuse.

10. Procédé selon l'une des revendications 7 à 9, caractérisé en ce que le polysaccharide précité est chauffé pendant une durée suffisante pour obtenir une dissolution totale du polysaccharide, puis la phase aqueuse est refroidie jusqu'à la température ambiante.

11. Procédé selon l'une des revendications 7 à 10, caractérisé en ce qu'on utilise comme phase organique une huile végétale, un ester d'acide gras, une huile minérale, un silicone ou un solvant organique apolaire, en particulier l'hexane, le cyclohexane ou le chloroforme.

12. Procédé selon l'une des revendication 7 à 11, caractérisé en ce qu'on réalise une émulsion en utilisant un agent émulsionnant dans une phase huileuse.

13. Procédé selon l'une des revendications 7 à 12, caractérisé en ce qu'on mélange intimement avec la phase aqueuse contenant le polysaccharide un actif hydrosoluble, hydrodispersible, insoluble ou liposoluble.

14. Procédé selon la revendication 13, caractérisé en ce que, lorsqu'il s'agit d'un actif liposoluble, on utilise un agent stabilisateur d'émulsion, par exemple un colloïde, une macromolécule ou un émulsionnant synthétique ou naturel.

15. Procédé selon l'une des revendications 7 à 14, caractérisé en ce que la proportion en poids de l'agent réticulant par rapport au poids de la solution polysaccharidique varie entre 20 et 60%.

16. Procédé selon l'une des revendications 7 à 15, caractérisé en ce que le polysaccharide précité est choisi parmi un polysaccharide naturel, par exemple les galactomannanes, les carraghénanes, les glucomannanes, les gommes naturelles, l'amylose ou l'amylopectine et leurs mélanges, les polysaccharides issus de fermentation ; ou un polysaccharide chimiquement modifié comprenant des groupements alcools primaires espacés de la chaîne de polymère plus aisément accessibles notamment à une éventuelle réaction avec un agent de réticulation, en particulier des dérivés hydroxyalkyles en C₁-C₃₀ de polysaccharides.

17. Composition alimentaire, cosmétique ou pharmaceutique, caractérisée en ce qu'elle comprend des microcapsules ou microsphères à paroi en polysaccharide à fonctions alcools primaires réticulées par un agent de réticulation constitué par un dichlorure d'acide ou un anhydride d'acide en formant ainsi des liaisons ester, lesdites microcapsules ou microsphères, de préférence, contiennent au moins en partie une substance présentant un intérêt alimentaire, cosmétique ou pharmaceutique.

18. Procédé de préparation d'une composition alimentaire, cosmétique ou pharmaceutique, caractérisé en ce qu'on utilise au moins en partie des microcapsules ou microsphères à paroi en polysaccharide ayant des fonctions alcools primaires réticulées par un agent de réticulation constitué par un dichlorure d'acide ou un anhydride d'acide en formant ainsi des liaisons ester, lesdites microcapsules ou microsphères contenant de préférence au moins en partie un principe actif alimentaire, cosmétique ou pharmaceutique.

## Claims

1. Use of polysaccharides having primary alcohol functions for the production of microcapsules or microspheres by interfacial crosslinking of the primary alcohol functions of said polysaccharide using an interfacial crosslinking agent constituted by an acid dichloride or an acid anhydride.

2. Microcapsules or microspheres, characterized in that they comprise a wall in polysaccharide having primary alcohol functions and being crosslinked by interfacial crosslinking using an interfacial crosslinking agent constituted by an acid dichloride or an acid anhydride.

3. Microcapsules or microspheres according to claim 2, characterized in that said polysaccharide has a molecular weight higher than 5,000 Daltons.

4. Microcapsules or microspheres according to claim 2 or 3, characterized in that said polysaccharide comprises between 1 and 4 primary alcohol functions per diosidic moiety.

5. Microcapsules or microspheres according to one of claims 2 to 4, characterized in that said polysaccharide is selected from a natural polysaccharide, such as galactomannans, carrageenans, glucomannans, natural gums, amylose or amylopectine and mixtures thereof, the polysaccharides issued from fermentation; or a chemically modified polysaccharide comprising primary alcohol groups spaced from the polymer chain and more readily accessible notably to a possible reaction with a crosslinking agent, in particular C₁-C₃₀ hydroaxylalkyl derivatives of polysaccharides.

6. Microcapsules or microspheres according to one of claims 2 to 5, characterized in that they contain a food, cosmetic or pharmaceutical active ingredient.

7. Method for producing microcapsules or microspheres, characterized in that it comprises the following steps:
- dispersing or dissolving in aqueous phase a polysaccharide having primary alcohol functions then obtaining an aqueous phase containing said polysaccharide in dispersion or in dissolved state, placing said aqueous phase in contact with an organic phase non-miscible in water and containing an interfacial crosslinking agent constituted by an acid dichloride or an acid anhydride, in order to produce an interfacial crosslinking of said primary alcohol functions of the polysaccharide for a long enough period to obtain microspheres or microcapsules with a wall comprising said polysaccharide having primary alcohol functions and being crosslinked by the acid dichloride or the acid anhydride thus forming ester bonds and said microspheres or microcapsules are then separated from the reaction medium by any separation means.

8. Method according to claim 7, characterized in that an alkaline aqueous phase is used, which has a pH higher than 7, in particular comprised between about 7.1 and about 10.

9. Method according to one of claims 7 to 8, characterized in that said polysaccharide is present in the aqueous phase at a concentration comprised between 0.2 % and 30 % by weight of said aqueous phase.

10. Method according to one of claims 7 to 9, characterized in that said polysaccharide is heated for a long enough period to obtain the total dissolution of the polysaccharide, then the aqueous phase is cooled down to room temperature.

11. Method according to one of claims 7 to 10, characterized in that the organic phase used is a vegetable oil, a fatty acid ester, a mineral oil, a silicone or an apolar organic solvent, in particular hexane, cyclohexane or chloroform.

12. Method according to one of claims 7 to 11, characterized in that an emulsion is prepared by using an emulsifying agent in an oily phase.

13. Method according to one of claims 7 to 12, characterized in that an active ingredient which is water-soluble, water-dispersible, non-soluble or liposoluble is intimately mixed with the aqueous phase containing the polysaccharide.

14. Method according to claim 13, characterized in that, in the case of a liposoluble active ingredient, an emulsion-stabilizing agent is used, for example a colloid, a macromolecule or a synthetic or natural emulsifier.

15. Method according to one of claims 7 to 14, characterized in that the proportion by weight of the crosslinking agent with respect to the weight of the polysaccharide-based solution varies between 20 and 60 %.

16. Method according to one of claims 7 to 15, characterized in that said polysaccharide is selected among a natural polysaccharide, for example galactomannans, carrageenans, glucomannans, natural gums, amylose or amylopectin and mixtures thereof, the polysaccharides issued from fermentation; or a chemically modified polysaccharide comprising primary alcohol groups spaced from the polymer chain and more readily accessible notably to a possible reaction with a crosslinking agent, in particular C₁-C₃₀ hydroxyalkyl derivatives of polysaccharides.

17. Food, cosmetic or pharmaceutical composition, characterized in that it comprises microcapsules or microspheres with a wall in polysaccharide with primary alcohol functions crosslinked by a crosslinking agent constituted by an acid dichloride or an acid anhydride thus forming ester bonds, said microcapsules or microspheres preferably contain at least partly a substance presenting a food, cosmetic or pharmaceutical advantage.

18. Method for the preparation of a food, cosmetic or pharmaceutical composition, characterized in that it comprises using at least partly microcapsules or microspheres with a wall in polysaccharide having primary alcohol functions crosslinked by a crosslinking agent constituted by an acid dichloride or an acid anhydride thus forming ester bonds, said microcapsules or microspheres preferably containing at least partly a food, cosmetic or pharmaceutical active ingredient.

## Patentansprüche

1. Verwendung von Polysacchariden mit primären Alkohol-Funktionen zur Herstellung von Mikrokapseln oder Mikrosphären durch Grenzflächenvernetzung der primären Alkohol-Funktionen des Polysaccharids mit einem Grenzflächenvernetzungsmittel, das aus einem Säuredichlorid oder einem Säureanhydrid besteht.

2. Mikrokapseln oder Mikrosphären, dadurch gekennzeichnet, daß sie eine Wand aus Polysaccharid mit primären Alkohol-Funktionen, die durch Grenzflächenvernetzung mit einem aus einem Säuredichlorid oder einem Säureanhydrid bestehenden Grenzflächenvernetzungsmittel vernetzt sind, umfassen.

3. Mikrokapseln oder Mikrosphären nach Anspruch 2, dadurch gekennzeichnet, daß das Polysaccharid eine Molmasse von mehr als 5000 Dalton aufweist.

4. Mikrokapseln oder Mikrosphären nach Anspruch 2 oder 3, dadurch gekennzeichnet, daß das Polysaccharid 1 bis 4 primäre Alkohol-Funktionen pro Diose-Einheit umfaßt.

5. Mikrokapseln oder Mikrosphären nach einem der Ansprüche 2 bis 4, dadurch gekennzeichnet, daß das Polysaccharid ausgewählt ist aus einem natürlichen Polysaccharid, beispielsweise Galactomannanen, Carrageenanen, Glucomannanen, natürlichen Gummen, Amylose oder Amylopektin und ihren Mischungen, aus einer Fermentation stammenden Polysacchariden; oder einem chemisch modifizierten Polysaccharid, das primäre Alkohol-Gruppen umfaßt, die in einem Abstand von der Polymer-Kette vorliegen, und die spezifisch bei einem möglichen Umsetzen mit einem Vernetzungsmittel leichter zugänglich sind, insbesondere C₁-C₃₀-Hydroxyalkyl-Derivaten von Polysacchariden.

6. Mikrokapseln oder Mikrosphären nach einem der Ansprüche 2 bis 5, dadurch gekennzeichnet, daß sie einen alimentären, kosmetischen oder pharmazeutischen aktiven Bestandteil enthalten.

7. Verfahren zur Herstellung von Mikrokapseln oder Mikrosphären, dadurch gekennzeichnet, daß es die folgenden Schritte umfaßt:
ein Polysaccharid mit primären Alkohol-Funktionen wird in einer wässerigen Phase dispergiert oder gelöst, indem eine wässerige Phase erhalten wird, die das Polysaccharid in Dispersion oder im gelösten Zustand enthält, die wässerige Phase wird mit einer organischen Phase, die mit Wasser nicht mischbar ist und ein aus einem Säuredichlorid oder einem Säureanhydrid bestehendes Grenzflächenvernetzungsmittel enthält, in Berührung gebracht, wobei eine Grenzflächenvernetzung der primären Alkohol-Funktionen des Polysaccharids während einer ausreichenden Dauer bewirkt wird, um Mikrosphären oder Mikrokapseln mit einer Wand zu erhalten, die das Polysaccharid mit durch das Säuredichlorid oder das Säureanhydrid vernetzten, primären Alkohol-Funktionen enthält, wobei so Ester-Bindungen gebildet werden, und anschließend werden die Mikrosphären oder Mikrokapseln vom Reaktionsmedium durch ein beliebiges Trennmittel getrennt.

8. Verfahren nach Anspruch 7, dadurch gekennzeichnet, daß eine alkalische wässerige Phase mit einem pH von mehr als 7, insbesondere zwischen etwa 7,1 und etwa 10, verwendet wird.

9. Verfahren nach einem der Ansprüche 7 oder 8, dadurch gekennzeichnet, daß das Polysaccharid in der wässerigen Phase in einer Konzentration zwischen 0,2 % und 30 %, bezogen auf die Masse der wässerigen Phase, vorliegt.

10. Verfahren nach einem der Ansprüche 7 bis 9, dadurch gekennzeichnet, daß das Polysaccharid während einer ausreichenden Dauer erhitzt wird, um ein vollständiges Lösen des Polysaccharids zu erhalten, und dann die wässerige Phase auf Umgebungstemperatur abgekühlt wird.

11. Verfahren nach einem der Ansprüche 7 bis 10, dadurch gekennzeichnet, daß als organische Phase ein Pflanzenöl, ein Fettsäureester, ein Mineralöl, ein Silikon oder ein apolares organisches Lösungsmittel, insbesondere Hexan, Cyclohexan oder Chloroform, verwendet wird.

12. Verfahren nach einem der Ansprüche 7 bis 11, dadurch gekennzeichnet, daß eine Emulsion unter Verwendung eines Emulgators in einer Ölphase hergestellt wird.

13. Verfahren nach einem der Ansprüche 7 bis 12, dadurch gekennzeichnet, daß ein hydrolöslicher, hydrodispergierbarer, unlöslicher oder fettlöslicher aktiver Bestandteil mit der wässerigen Phase, die das Polysaccharid enthält, innig gemischt wird.

14. Verfahren nach Anspruch 13, dadurch gekennzeichnet, daß, wenn es sich um einen fettlöslichen aktiven Bestandteil handelt, ein Emulsionsstabilisator, beispielsweise ein Kolloid, ein Makromolekül oder ein synthetischer oder natürlicher Emulgator, verwendet wird.

15. Verfahren nach einem der Ansprüche 7 bis 14, dadurch gekennzeichnet, daß die Massemenge des Vernetzungsmittels in bezug auf die Masse der Polysaccharid-Lösung zwischen 20 und 60 % variiert.

16. Verfahren nach einem der Ansprüche 7 bis 15, dadurch gekennzeichnet, daß das Polysaccharid ausgewählt wird aus einem natürlichen Polysaccharid, beispielsweise Galactomannanen, Carrageenanen, Glucomannanen, natürlichen Gummen, Amylose oder Amylopektin und ihren Mischungen, aus einer Fermentation stammenden Polysacchariden; oder einem chemisch modifizierten Polysaccharid, das primäre Alkohol-Gruppen umfaßt, die in einem Abstand von der Polymer-Kette vorliegen, und die spezifisch bei einem möglichen Umsetzen mit einem Vernetzungsmittel leichter zugänglich sind, insbesondere C₁-C₃₀Hydroxyalkyl-Derivaten von Polysacchariden.

17. Alimentäre, kosmetische oder pharmazeutische Zusammensetzung, dadurch gekennzeichnet, daß sie Mikrokapseln oder Mikrosphären mit einer Wand aus Polysaccharid mit primären Alkohol-Funktionen, die durch Grenzflächenvernetzung mit einem aus einem Säuredichlorid oder einem Säureanhydrid bestehenden Grenzflächenvernetzungsmittel vernetzt sind, umfaßt, wobei so Ester-Bindungen gebildet werden, welche Mikrokapseln oder Mikrosphären vorzugsweise zumindest teilweise eine Substanz von alimentärem, kosmetischen oder pharmazeutischem Interesse enthalten.

18. Verfahren zur Herstellung einer alimentären, kosmetischen oder pharmazeutischen Zusammensetzung, dadurch gekennzeichnet, daß zumindest teilweise Mikrokapseln oder Mikrosphären mit einer Wand aus Polysaccharid mit primären Alkohol-Funktionen, die mit einem aus einem Säuredichlorid oder einem Säureanhydrid bestehenden Grenzflächenvernetzungsmittel vernetzt sind, verwendet werden, welche Mikrokapseln oder Mikrosphären vorzugsweise zumindest teilweise einen alimentären, kosmetischen oder pharmazeutischen aktiven Bestandteil enthalten.
